# EUROPEAN PATENT SPECIFICATION

(11) **EP 2 319 389 B1**
(45) Date of publication and mention of the grant of the patent: **26.02.2014**
(21) Application number: 10015395.6
(22) Date of filing: 03.04.2009
(51) Int. Cl.: A61B 1/05, A61B 5/00, A61B 5/07, H01Q 1/08

(54) **Antenna unit and receiving apparatus for capsule medical apparatus**
Antenneneinheit und Empfangsvorrichtung für medizinische Kapselvorrichtung
Unité d'antenne et appareil de réception pour appareil médical à capsule

(30) Priority: 03.04.2008 JP 2008097303; 22.05.2008 JP 2008134176
(43) Date of publication of application: 11.05.2011
(62) Divisional of application: 09004992.5
(73) Proprietor: Olympus Medical Systems Corporation, Shibuya-ku Tokyo 151-0072 (JP)
(72) Inventor: Takenaka, Tomoya, Tokyo 151-0072 (JP); Homan, Masatoshi, Tokyo 151-0072 (JP); Kimoto, Seiichiro, Tokyo 151-0072 (JP)
(74) Representative: Frenkel, Matthias Alexander

(56) References cited:
- WO-A-03/065926
- WO-A-2005/004033
- US-A1- 2006 121 851
- ABAD E ET AL: "Investigation of fabrication and encapsulation processes for a flexible tag microlab", MICROSYSTEM TECHNOLOGIES ; MICRO AND NANOSYSTEMS INFORMATION STORAGE AND PROCESSING SYSTEMS, SPRINGER, BERLIN, DE, vol. 14, no. 4-5, 18 October 2007 (2007-10-18), pages 527-534, XP019591713, ISSN: 1432-1858

## Description

### TECHNICAL FIELD

The present invention relates to an antenna unit which is arranged on a subject, e.g., a patient and which relays image data transmitted wirelessly from a capsule medical apparatus within the subject to a receiving apparatus for a capsule medical apparatus (hereinafter, "medical receiving apparatus") located outside the subject. The present invention also relates to the above-mentioned medical receiving apparatus, which is integrated with receiving antennas for receiving the image data transmitted wirelessly from the capsule medical apparatus.

### BACKGROUND ART

In conventional endoscopy technology, swallowable capsule medical apparatuses which have imaging and wireless transmission functions have been proposed. Such an apparatus is swallowed by a subject for observation (examination) purposes. The apparatus sequentially captures intracelomic images of the subject using its imaging function while moving through the body cavity, e.g., organs such as the stomach and the small intestine, due to peristalsis. It continuously takes images until naturally excreted. The images of the subject's organs taken inside the body are also referred to below as in-vivo images. Image data of the captured in-vivo images are sequentially transmitted using the wireless transmission function to a medical receiving apparatus which is located outside the subject. The medical receiving apparatus receives the transmitted image data via receiving antennas, performs a predetermined process on the image data, and sequentially stores the processed image data in a recording medium.

An external receiver has been proposed in which more than one receivers are arranged on an outer abdominal surface or an outer dorsal surface of a subject to pick up a wireless signal containing in-vivo images from a capsule medical apparatus inside the subject (see, Japanese Patent Application Laid-Open No. 2007-82664). The external receiver disclosed in Japanese Patent Application Laid-Open No. 2007-82664 receives an image signal transmitted from the capsule endoscope inside a subject, and transmits the received image signal to an image storage apparatus.

Wireless signals from the capsule medical apparatus inside the subject and received by the receiving antennas incorporated in the medical receiving apparatus are faint. Therefore, it is preferable that the receiving antennas be located near the capsule medical apparatus. To achieve this, receiving antennas made of a flexible substrate are located on the subject for example.

In contrast, a main unit of the medical receiving apparatus contains parts which are not flexible: a lithium secondary battery, which is a power source; and an LCD, which is a display device for displaying various pieces of information. Therefore, the battery and the LCD are carried by the subject in a small shoulder bag or the like. The medical receiving apparatus, which has receiving, signal processing, and storage functions, is carried by the subject when in use. Thus, between when the capsule medical apparatus is swallowed and when it is naturally excreted, the subject can act freely (for example, see Japanese Patent Application Laid-Open No. 2006-551431).

For examples of a sheet-shaped display device which works as a display device for a personal computer, see Japanese Patent Application Laid-Open No. 2005-316672, and No. 2006-30718.

The recording medium, which stores therein the image data of the in-vivo image as described, is detached from the medical receiving apparatus when the capsule medical apparatus is naturally excreted by the subject. The recording medium is then mounted on a predetermined image display device. The image display device loads a series of image data in the recording medium, i.e., a series of in-vivo images of the subject which were captured by the capsule medical apparatus, and displays the series of in-vivo images on the display. A user such as a doctor and a nurse observes the series of in-vivo images being displayed on the image display device, and can thus diagnose the subject.

The receiving antenna, which receives the wireless signals from the capsule medical apparatus inside the subject as described, is connected with the receiving apparatus located outside the subject. The connection is via a cable. The antenna units transmit the wireless signal from the capsule medical apparatus via the cable to the receiving apparatus. Because the receiving apparatus is connected via the cable, the receiving antennas arranged on the subject cannot be freely positioned or directed. This makes attaching the receiving antenna to the subject a difficult task. Further, when the subject moves, he/she has to be always aware of the cable which connects the receiving antenna with the receiving apparatus.

For systems for obtaining the in-vivo information such as in-vivo images using the capsule medical apparatus inside the subject, there is demand for antenna units which relay the wireless signal from the capsule medical apparatus to the receiving apparatus outside the subject and, at the same time, can be easily attached to the subject so that the burden on the subject is reduced.

In the medical receiving apparatus described above, the antenna is separate from the receiving-apparatus main unit and is connected with the same via a cable and a coaxial cable, which results in a loss of power in reception, and a reduction of resistance to external noise.

WO 2005/004033 A relates to a system and method for receiving an in vivo signal, using for example a receiver, a recorder and an antenna array. The receiver may include for example a switching unit and an amplifier, and may be in proximity to the antenna array.

WO 03/065926 A relates to a sensor system which has a sensor module and a receiver module. The sensor module functions as a wireless data collection device and has a flexible thin sheet of silicon comprising circuitry, a flexible power source, and a flexible support substrate. The silicon, power source, and flexible support substrate are integrated as layers of the sensor module. The layers are placed together in the form of an adhesive bandage. A plurality of electrodes are connected to the sensor module and protrude from the flexible substrate for contacting the skin of a subject body. The receiver module includes one of an RF receiver with a wireless port for continuously receiving data, or a physical I/O port to which the sensor module can be physically connected for downloading stored data from the sensor module.

### DISCLOSURE OF INVENTION

A receiving apparatus for a capsule medical apparatus according to the present invention includes the features of independent claim 1. Preferred embodiments are defined by the dependent claims.

The above and other features, advantages and technical and industrial significance of this invention will be better understood by reading the following detailed description of presently preferred embodiments of the invention, when considered in connection with the accompanying drawings.

### BRIEF DESCRIPTION OF DRAWINGS

FIG. 1 is a schematic diagram of an exemplary configuration of an in-vivo information obtaining system which includes an antenna unit according to a first embodiment.
FIG. 2 is a schematic diagram of an exemplary configuration of the antenna unit according to the first embodiment.
FIG. 3 is a side-elevation schematic diagram which views the antenna unit shown in FIG. 2, viewed in direction A.
FIG. 4 is a schematic block diagram of an exemplary functional configuration of the antenna unit according to the first embodiment.
FIG. 5 is a schematic diagram of an example configuration of the antenna unit according to a second embodiment.
FIG. 6 is a cross-sectional schematic diagram of the antenna unit shown in FIG. 5, taken along line B-B.
FIG. 7 is a schematic diagram of an illustration of a layer structure of a battery content of the antenna unit according to the second embodiment.
FIG. 8 is a schematic diagram of an exemplary configuration of an antenna unit according to a third embodiment.
FIG. 9 is a side-elevation schematic diagram which views the antenna unit shown in FIG. 8, viewd in direction A.
FIG. 10 is a schematic diagram of an exemplary configuration of an antenna unit according to a fourth embodiment.
FIG. 11 is a schematic block diagram of an exemplary functional configuration of the antenna unit according to the fourth embodiment.
FIG. 12 is a configuration diagram of a medical receiving apparatus according to a fifth embodiment to which the present invention is applied.
FIG. 13 is a cross-sectional view of a side surface of the medical receiving apparatus according to the fifth embodiment to which the present invention is applied.
FIG. 14A shows a flexible display device according to the fifth embodiment to which the present invention is applied.
FIG. 14B shows a flexible receiving-apparatus main unit according to the fifth embodiment to which the present invention is applied.
FIG. 14C shows a flexible power apparatus according to the fifth embodiment to which the present invention is applied.
FIG. 14D shows a flexible antenna apparatus according to the fifth embodiment to which the present invention is applied.
FIG. 14E shows a flexible adhesive sheet according to the fifth embodiment to which the present invention is applied.
FIG. 15 is a cross-sectional view of a side surface of a medical receiving apparatus according to a sixth embodiment to which the present invention is applied.
FIG. 16 shows a flexible receiving-apparatus main unit according to the sixth embodiment to which the present invention is applied.
FIG. 17 is a cross-sectional view of a side surface of a medical receiving apparatus according to a seventh embodiment to which the present invention is applied.
FIG. 18A shows a flexible display device according to the seventh embodiment to which the present invention is applied.
FIG. 18B shows a flexible receiving-apparatus main unit according to the seventh embodiment to which the present invention is applied.
FIG. 18C shows a flexible power apparatus according to the seventh embodiment to which the present invention is applied.
FIG. 18D shows a flexible antenna apparatus according to the seventh embodiment to which the present invention is applied.
FIG. 18E shows a flexible adhesive sheet according to the seventh embodiment to which the present invention is applied.

### BEST MODE(S) FOR CARRYING OUT THE INVENTION

Exemplary embodiments of an antenna unit forming a part of the present invention are described in detail with reference to the accompanying drawings. An example of such an antenna unit according to the present invention is described below. The antenna unit relays in-vivo images, which are an example of in-vivo information of a subject, captured by a capsule medical apparatus inside the subject to a medical receiving apparatus outside the subject. The in-vivo images are an example of in-vivo information of a subject. The present invention is not limited to the embodiments.

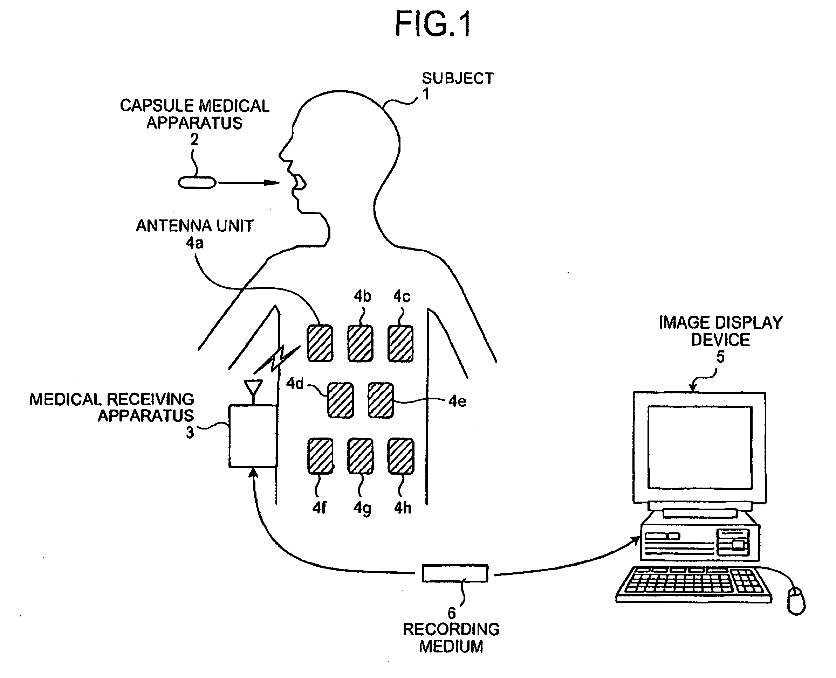

FIG. 1 is a schematic diagram of an exemplary configuration of an in-vivo information obtaining system which includes an antenna unit according to a first embodiment. As used herein, "antenna unit" is sometimes intended to encompass a plurality of antenna units. The in-vivo information obtaining system obtains the in-vivo images, which are an example of the in-vivo information of a subject 1. As shown in FIG. 1, the in-vivo information obtaining system includes a capsule medical apparatus 2, a medical receiving apparatus 3, more than one antenna units 4a to 4h, an image display device 5, and a recording medium 6. The capsule medical apparatus 2 captures an in-vivo image of the subject 1. The medical receiving apparatus 3 forming the invention receives the in-vivo image taken inside the subject 1. The antenna units 4a to 4h relay wireless signals from the capsule medical apparatus 2 inside the subject 1 to the medical receiving apparatus 3 outside the subject 1. The image display device 5 displays various pieces of information, e.g., the in-vivo images of the subject 1. The recording medium 6 is used for exchanging data between the medical receiving apparatus 3 and the image display device 5.

The capsule medical apparatus 2 is a capsule-type, medical apparatus which has imaging and wireless transmission functions. Specifically, the capsule medical apparatus 2 is introduced inside the subject 1 through oral intake or the like, and moves through the digestive tracts of the subject 1 due to peristalsis or the like until it is naturally excreted. Until the capsule medical apparatus 2 introduced inside the subject 1 is excreted outside the subject 1, the capsule medical apparatus 2 sequentially captures the in-vivo images of the subject 1 at predetermined intervals, e.g., 0.5-second intervals, and wirelessly sequentially transmits the image signals of the obtained in-vivo images to the outside of the subject 1.

The medical receiving apparatus 3 forming the invention receives and stores a series of the in-vivo images of the subject 1. Specifically, the medical receiving apparatus 3 is a portable apparatus which is carried by the subject 1 who has had the capsule medical apparatus 2 introduced inside his/her body. The medical receiving apparatus 3 includes a receiving antenna for wireless transmission with at least one of the antenna units 4a to 4h. The medical receiving apparatus 3 receives the wireless signals from the capsule medical apparatus 2 inside the subject 1 via the antenna units 4a to 4h. In each reception of the wireless signal, the medical receiving apparatus 3 obtains the image data, i.e., the in-vivo image of the subject 1 captured by the capsule medical apparatus 2 out of the received wireless signal. The medical receiving apparatus 3 stores the obtained series of in-vivo images of the subject 1 in the recording medium 6. The recording medium 6 is detachable from the medical receiving apparatus 3.

After the capsule medical apparatus 2 is introduced inside the subject 1, it takes a relatively long time, e.g., a few hours for the medical receiving apparatus 3 to receive a complete series of in-vivo images for observation taken in organs which are "deep" (far from the mouth) within the body, e.g., inside the entire digestive tract or the small and large intestines of the subject 1. Therefore, as shown in FIG. 1, the medical receiving apparatus 3 is actually carried by the subject 1. Sometimes, it may be unnecessary to carry the medical receiving apparatus 3 when receiving the in-vivo images near the mouth cavity, e.g., in the esophagus, or stomach. In this case, the medical receiving apparatus 3 may be positioned within a predetermined distance from the subject 1, e.g., in the same room as the subject 1. With the medical receiving apparatus 3 carried or positioned as described above, the subject 1 can move freely until the medical receiving apparatus 3 completes the obtainment of the series of in-vivo images of the subject 1.

The antenna units 4a to 4h are spread out over the subject 1 who the capsule medical apparatus 2 is introduced inside. The antenna units 4a to 4h relay the in-vivo image of the subject 1 obtained by the capsule medical apparatus 2 inside the subject 1 to the external medical receiving apparatus 3. The antenna units 4a to 4h are spread out and attached to the body surface of the subject 1 with an adhesive material or the like. Each of the antenna units 4a to 4h relays the wireless signals of the in-vivo image transmitted from the capsule medical apparatus 2 inside the subject 1 to the external medical receiving apparatus 3. Each of the antenna units 4a to 4h receives the wireless signal transmitted from the capsule medical apparatus 2. The antenna units 4a to 4h process the received wireless signals in a predetermined way and wirelessly transmit the wireless signals, which include the in-vivo images of the subject 1 on which the signal processing has been performed, to the external medical receiving apparatus 3.

The antenna units 4a to 4h may be attached to different parts of a jacket or the like worn by the subject 1. Because the jacket is worn by the subject 1, the antenna units 4a to 4h are spread out over the body surface of the subject 1. Further, the number of the arranged antenna units may be one or more and is not limited to eight.

The image display device 5 has a configuration similar to a workstation and displays various pieces of information, e.g., in-vivo images of the subject 1 captured by the capsule medical apparatus 2. Specifically, the recording medium 6 is detached from the medical receiving apparatus 3, and attached to the image display device 5, and the series of in-vivo images and the like of the subject 1 stored in the recording medium 6 are read out by the image display device 5. The image display device 5 then displays the series of the in-vivo images of the subject 1 on a display according to instructions from the users, e.g., doctors and nurses. Further, the image display device 5 has a processing function for diagnosing problems the subject 1 has with reference to the in-vivo images of the subject 1.

The recording medium 6 is a portable recording medium and is used for exchanging data between the medical receiving apparatus 3 and the image display device 5 described above. Specifically, the recording medium 6 can be attached to and detached from the medical receiving apparatus 3 and the image display device 5, and can output or store data when attached to either of them. When attached to the medical receiving apparatus 3, the recording medium 6 stores the series of the in-vivo images received by the medical receiving apparatus 3, whereas when it is attached to the image display device 5, the recording medium 6 outputs stored data such as the in-vivo image and the like of the subject 1 to the image display device 5.

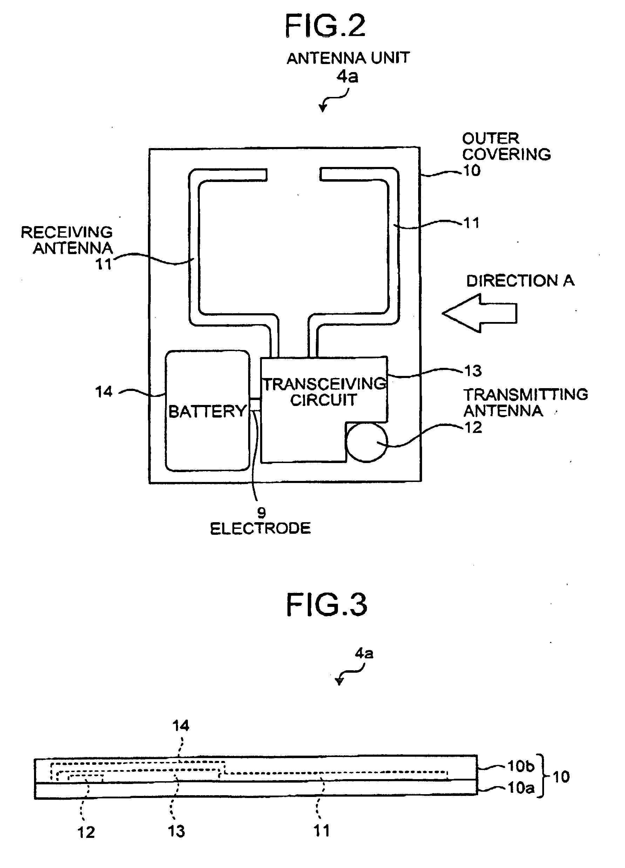

A configuration of the antenna units 4a to 4h according to the first embodiment is described. FIG. 2 is a schematic diagram of an exemplary configuration of the antenna unit according to the first embodiment. FIG. 3 is a schematic diagram of a side surface of the antenna unit shown in FIG. 2, viewed in direction A. In FIG. 2, an upper layer of an outer covering 10, i.e., a covering 10b described later, is omitted so that inner parts of the antenna unit 4a can be more easily illustrated. The following describes only the antenna unit 4a of the antenna units 4a to 4h. The remaining antenna units 4b to 4h have the same configuration as the antenna unit 4a.

As shown in FIGS. 2 and 3, the antenna unit 4a includes a flexible outer covering 10, a receiving antenna 11, a transmitting antenna 12, a transceiving circuit 13, and a battery 14. The receiving antenna 11 receives the in-vivo image transmitted wirelessly from the capsule medical apparatus 2 inside the subject 1. The transmitting antenna 12 wirelessly transmits the in-vivo image received from the capsule medical apparatus 2 to the external medical receiving apparatus 3. The transceiving circuit 13 generates the wireless signal of the in-vivo image to be wirelessly transmitted via the transmitting antenna 12 to the medical receiving apparatus 3. The battery 14 supplies power for the transceiving circuit 13. The battery 14 may supply power for the transceiving circuit 13 directly, or supply power for the transceiving circuit 13 via a voltage generator (not shown) such as a regulator and a DC-DC converter.

The outer covering 10 is flexible, and carries each component of the antenna unit 4a, i.e., the receiving antenna 11, the transmitting antenna 12, the transceiving circuit 13, and the battery 14. The outer covering 10 is realized by a covering 10b and a flexible circuit substrate 10a which can be easily transformed according to external acting force. The flexible circuit substrate 10a is flexible, a circuit substrate, and made of a resin member such as polyimide. The flexible circuit substrate 10a previously includes circuits thereon which are required for realizing a function of the transceiving circuit 13. The receiving antenna 11, the transmitting antenna 12, the transceiving circuit 13, and the battery 14 are mounted on the flexible circuit substrate 10a. The receiving antenna and the transmitting antenna may be formed directly on the flexible circuit substrate 10a as a circuit pattern. The covering 10b is a flexible insulating member made of a resin such as polyvinyl chloride and polypropylene. The covering 10b is attached to the flexible circuit substrate 10a via a predetermined processing method, e.g., lamination. The covering 10b covers each of the components such as the receiving antenna 11, the transmitting antenna 12, the transceiving circuit 13, and the battery 14, which are mounted on or carried by the flexible circuit substrate 10a.

The outer covering 10, which is realized by the flexible circuit substrate 10a and the covering 10b, is flexible and can be transformed according to external acting force (e.g., force which is received from the body surface of the subject 1). The outer covering 10 contains the receiving antenna 11, the transmitting antenna 12, the transceiving circuit 13, and the battery 14. The outer covering 10 may have the adhesive member attached to an outer surface of the flexible circuit substrate 10a or the covering 10b so that the outer covering 10 can be detachably attached to the body surface of the subject 1.

The receiving antenna 11 is a flexible antenna which is made of a thin metal member such as a copper foil. The receiving antenna 11 receives the wireless signal from the capsule medical apparatus 2 inside the subject 1, and transmits the received wireless signal to the transceiving circuit 13. The transmitting antenna 12 transmits the wireless signal generated by the transceiving circuit 13 to the external medical receiving apparatus 3. When the transceiving circuit 13 transmits the wireless signal to the medical receiving apparatus 3 while receiving the wireless signal from the capsule medical apparatus 2, it is preferable that the wireless signal to the medical receiving apparatus 3 be transmitted at a different frequency from that of the wireless signal transmitted from the capsule medical apparatus 2. When the wireless signal to the medical receiving apparatus 3 is transmitted at a higher frequency than that of the wireless signal transmitted from the capsule medical apparatus 2, the transmitting antenna 12 can be downsized. Further, when the wireless signal to the medical receiving apparatus 3 is transmitted at the same frequency as that of the wireless signal transmitted from the capsule medical apparatus 2, it is preferable that a time-division transceiving method be adopted so that the transmission of the wireless signal to the medical receiving apparatus 3 is performed during a different period from the reception of the wireless signal from the capsule medical apparatus 2. The wireless signal, which is received by the receiving antenna 11 or transmitted from the transmitting antenna 12, includes the data of the in-vivo image of the subject captured by the capsule medical apparatus 2 described above.

The transceiving circuit 13 has a function as the wireless-signal generator which generates the wireless signal which includes the in-vivo image of the subject 1 received by the receiving antenna 11. The transceiving circuit 13 receives the wireless signal from the capsule medical apparatus 2 via the receiving antenna 11, and, in each reception, performs the predetermined transmission process on the received signal to generate the wireless signal which can be received by the external medical receiving apparatus 3. The wireless signal generated by the transceiving circuit 13 includes the data of the in-vivo image of the subject 1 captured by the capsule medical apparatus 2. The transceiving circuit 13 transmits the generated wireless signal to the external medical receiving apparatus 3 via the transmitting antenna 12.

The battery 14 has a function as the power supply unit which supplies power for the transceiving circuit 13 described above. The battery 14 is a sheet-shaped battery, and much thinner and lighter than a button-shaped battery, a dry-cell battery, and the like. The battery 14 is mounted on the flexible circuit substrate 10a of the outer covering 10 and supplies power for the transceiving circuit 13 via an electrode 9 when switched to an ON state by a switch unit (not shown). The battery 14 stops supplying power for the transceiving circuit 13 when switched to an OFF state by the switching unit. The power supply for the transceiving circuit 13 may be performed by the battery 14 directly or via the voltage generator such as the regulator and the DC-DC converter (not shown) arranged between the transceiving circuit 13 and the battery 14. The battery 14 is flexible and can be easily transformed according to transformation of the outer covering 10 due to external acting force. Specifically, the battery 14 can be easily transformed according to transformation of the outer covering 10 when the outer covering 10 is transformed according to movement of the subject 1.

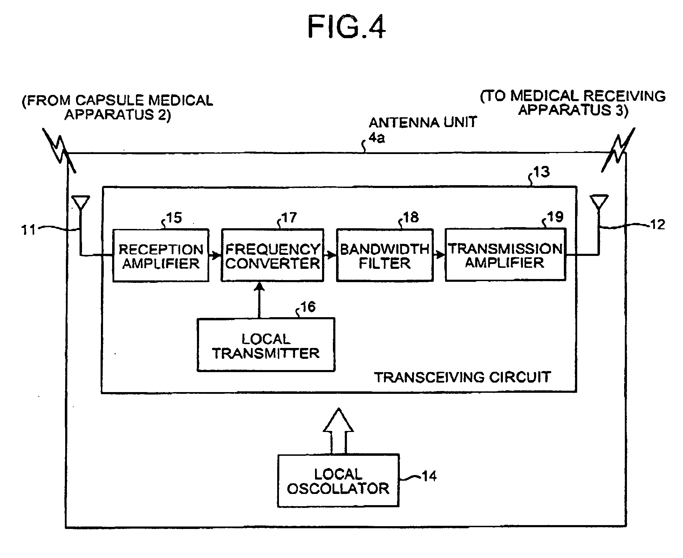

The transceiving circuit 13 of the antenna unit 4a according to the first embodiment is described below in detail. FIG. 4 is a schematic block diagram of an exemplary functional configuration of the antenna unit according to the first embodiment. As shown in FIG. 4, the transceiving circuit 13 of the antenna unit 4a includes a reception amplifier 15, a local oscillator 16, a frequency converter 17, a bandwidth filter 18, and a transmission amplifier 19. The reception amplifier 15 amplifies the received signal of the receiving antenna 11. The local oscillator 16 sends out predetermined high-frequency signals. The frequency converter 17 converts the frequency of the received signal transmitted from the capsule medical apparatus 2. The bandwidth filter 18 removes components of unnecessary frequency out of the wireless signal whose frequency has been converted. The transmission amplifier 19 amplifies the wireless signal transmitted to the medical receiving apparatus 3 via the transmitting antenna 12.

The reception amplifier 15 amplifies the wireless signal which is received by the receiving antenna 11 from the capsule medical apparatus 2, i.e., the image signal of the in-vivo image of the subject 1 captured by the capsule medical apparatus 2. The reception amplifier 15 transmits the amplified received signal to the frequency converter 17.

The frequency converter 17 generates the wireless signal which can be received by the external medical receiving apparatus 3, based on the wireless signal transmitted from the capsule medical apparatus 2 and the high-frequency signal from the local oscillator 16. Specifically, the frequency converter 17 mixes the received signal amplified by the reception amplifier, i.e., the wireless signal transmitted from the capsule medical apparatus 2 with the high-frequency signal from the local oscillator 16, and thus converts the received signal into the high-frequency signal. The wireless signal, which is generated by the frequency converter 17 which converts the received signal into the high-frequency signal, is a signal generated by converting the frequency of the wireless signal into the high frequency and can be received by the external medical receiving apparatus 3. The frequency converter 17 transmits the wireless signal whose frequency is converted into the high frequency to the bandwidth filter 18. When the signal is converted into the high-frequency signal by the frequency converter 17, the transmitting antenna 12 can be downsized.

The bandwidth filter 18 allows transmission of signal component within a predetermined bandwidth in the wireless signal and attenuates the unnecessary signal component outside the predetermined bandwidth for the signal component input from the frequency converter 17. The transmission amplifier 19 amplifies the wireless signal whose unnecessary signal component is removed by the bandwidth filter 18. The wireless signal which is amplified by the transmission amplifier 19 includes the data of the in-vivo image of the subject 1, and is transmitted to the external medical receiving apparatus 3 via the transmitting antenna 12, and then received by the medical receiving apparatus 3 described above.

As described above, in the first embodiment, mounted on the flexible circuit substrate, which is a part of the flexible outer covering which can be easily transformed according to external acting force, are the receiving antenna which receives the wireless signal from the capsule medical apparatus inside the subject, the transceiving circuit which converts the wireless signal received by the receiving antenna into the high-frequency signal, the transmitting antenna which transmits the wireless signal which has been converted into the high-frequency signal by the transceiving circuit to the external medical receiving apparatus, and the sheet-shaped battery which supplies power for the transceiving circuit. The battery is flexible and can be easily transformed according to the transformation of the outer covering. Thus, the antenna unit according to the first embodiment can relay the wireless signal from the capsule medical apparatus inside the subject to the external medical receiving apparatus without using a cable. The antenna unit can maintain flexibility and can be transformed according to the movement of the subject. Furthermore, the antenna unit is suitable for downsizing and reduction in thickness. It is not necessary to connect the antenna unit with the external medical receiving apparatus via the cable. The antenna unit is positioned independently of the external medical receiving apparatus or the cable and thus can be easily attached to a desired position of the subject. The antenna unit can avoid causing unpleasant feeling on the subject, which is often caused when the antenna unit is attached directly to his/her body surface. As a result, the antenna unit can reduce burden of the subject in receiving the in-vivo information obtained by the capsule medical apparatus inside the subject and can be easily arranged over the subject.

Because the antenna unit converts the wireless signal received from the capsule medical apparatus inside the subject into the high-frequency signal and relays the same to the external medical receiving apparatus, a circuit size of the transceiving circuit mounted on the antenna unit can be downsized and thereby the antenna unit can be further downsized and made lighter. Furthermore, because the wireless signal is converted into the high-frequency signal, the transmitting antenna which transmits the wireless signal to the external medical receiving apparatus can be downsized and thereby the antenna unit can be further downsized and made lighter.

A second embodiment is described below. In the first embodiment described above, the flexible circuit substrate 10a, which is a part (a circuit layer) of the flexible outer covering 10, has the receiving antenna 11, the transmitting antenna 12, the transceiving circuit 13, and the battery 14 mounted thereon. In contrast, in the second embodiment, a battery outer covering is used as the outer covering of the antenna unit, and the receiving antenna 11, the transmitting antenna 12, the transceiving circuit 13, and the like are mounted on a sealing member which forms the battery outer covering.

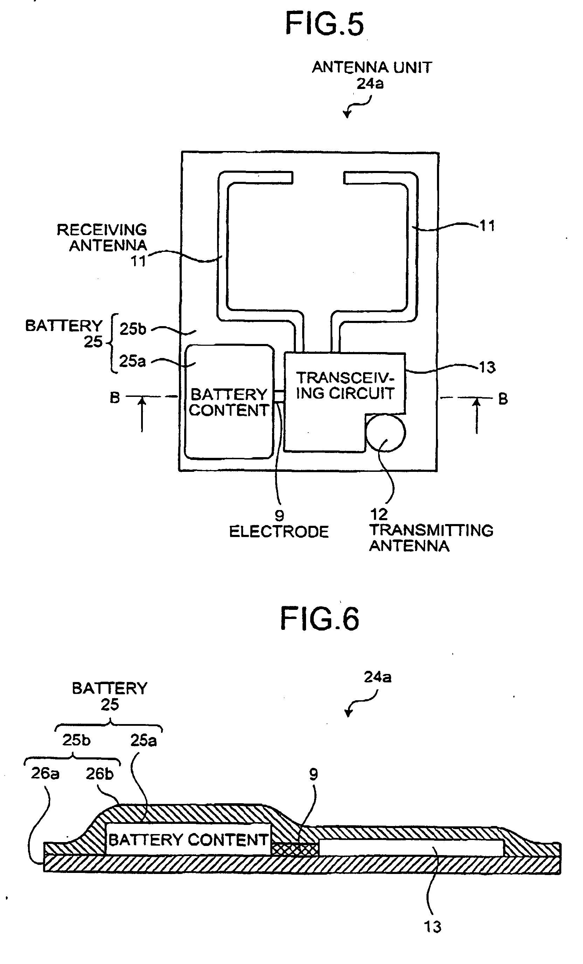

FIG. 5 is a schematic diagram of an exemplary configuration of the antenna unit according to the second embodiment. FIG. 6 is a cross-sectional schematic diagram of the antenna unit shown in FIG. 5 taken along line B-B. In FIG. 5, an upper layer of the outer covering, i.e., an upper-layer sealing member 26b shown in FIG. 6, is omitted so that the inner parts of the antenna unit can be more easily illustrated.

As shown in FIGS. 5 and 6, an antenna unit 24a according to the second embodiment includes a battery 25 instead of the battery 14 of the antenna unit 4a according to the first embodiment described above. A battery outer covering 25b, which is an outer covering of the battery 25, contains (seals) the battery content 25a and the like, and also works as the outer covering of the entire antenna unit 24a. Other configurations of the antenna unit 24a are the same as those in the first embodiment, and the same numerals are attached to the same components.

Although not shown in the figures, an in-vivo information obtaining system according to the second embodiment includes antenna units 24a to 24h instead of the antenna units 4a to 4h of the in-vivo information obtaining system according to the first embodiment shown in FIG. 1. Of the antenna units 24a to 24h, the antenna unit 24a is described below. The remaining antenna units 24b to 24h have the same configuration as the antenna unit 24a.

The battery 25 is a polymer battery, e.g., a lithium polymer battery, and includes the battery content 25a and the battery outer covering 25b which contains at least the battery content 25a. The battery content 25a includes a flexible member such as a gel electrolyte and has a function as the power supply unit which supplies power for the transceiving circuit 13 described above. The battery content 25a is connected with the transceiving circuit 13 via the electrode 9 which is arranged inside the battery outer covering 25b (specifically, above a lower-layer sealing member 26a described later). The battery content 25a contained in the battery outer covering 25b supplies power for the transceiving circuit 13 via the electrode 9 when switched to an ON state by the switch unit (not shown). The battery content 25a stops supplying power for the transceiving circuit 13 when switched to an OFF state by the switch unit. The power may be supplied from the battery 25 for the transceiving circuit 13 directly or via the voltage generator such as the regulator and the DC-DC converter (not shown) arranged between the transceiving circuit 13 and the battery 25.

The battery outer covering 25b is realized by a pair of the lower-layer sealing member 26a and the upper-layer sealing member 26b which contain at least the battery content 25a. The lower-layer sealing member 26a is a sealing member which forms a lower layer of the outer covering of the entire antenna unit 24a. Circuits which are needed for realizing the function of the transceiving circuit 13 are previously mounted on the lower-layer sealing member 26a, instead of the flexible circuit substrate 10a of the antenna unit 4a according to the first embodiment described above. The lower-layer sealing member 26a has the receiving antenna 11, the transmitting antenna 12, and the transceiving circuit 13 above mounted thereon. The lower-layer sealing member, on which the components of the antenna unit 24a are mounted, is made of a soft resin member, and works as a flexible circuit substrate which can be transformed easily according to the external acting force, similarly to the flexible circuit substrate 10a described above. The resin member which forms the lower-layer sealing member 26a is, for example, polyimide, and polyether nitrile.

The upper-layer sealing member 26b is a sealing member which is a replacement for the covering 10b of the antenna unit 4a according to the first embodiment described above. The upper-layer sealing member 26b forms an upper layer of the outer covering of the entire antenna unit 24a. The upper-layer sealing member 26b is made of a soft resin member and covers the components (the battery content 25a, the receiving antenna 11, the transmitting antenna 12, and the transceiving circuit 13) which are mounted on the lower-layer sealing member 26a. The resin member which forms the upper-layer sealing member 26b is, for example, polyvinyl chloride, polypropylene, polyethylene, polyethylene terephthalate, polyester, and polyolefin.

The battery outer covering 25b which is realized by the flexible lower-layer sealing member 26a and the upper-layer sealing member 26b is flexible and can be transformed easily according to the external acting force (e.g., force received from the body surface oft the subject 1). The battery outer covering 25b contains (seals) the receiving antenna 11, the transmitting antenna 12, the transceiving circuit 13, and the battery content 25a. Further, the battery outer covering 25b may have the adhesive member on the outer surface of the lower-layer sealing member 26a or the upper-layer sealing member 26b so that the battery outer covering 25b can be detachably attached to the body surface of the subject 1.

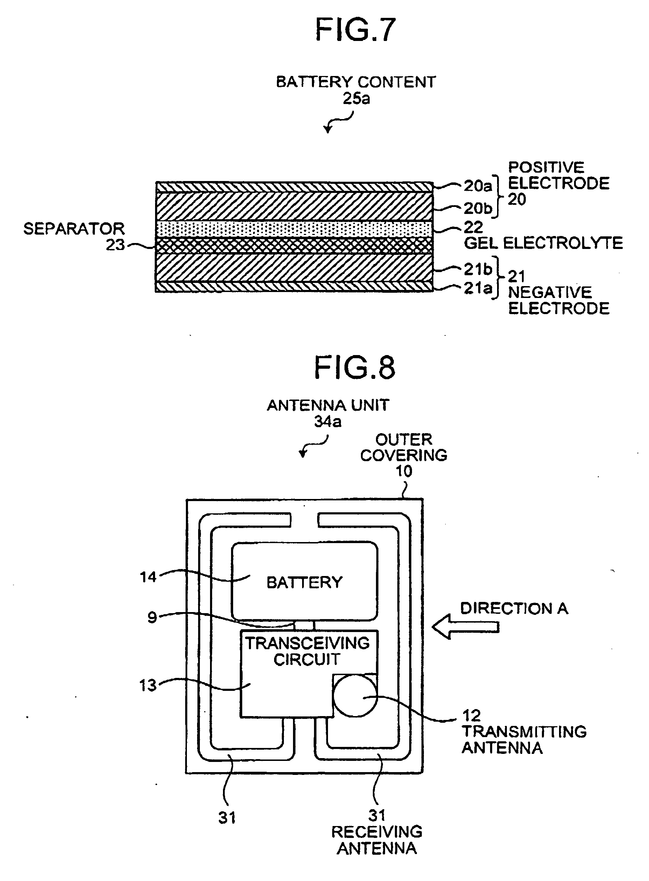

The battery content 25a contained in the above-described battery outer covering 25b is described. FIG. 7 is a schematic diagram of an illustration of a layer structure of the battery content of the antenna unit according to the second embodiment. As shown in FIG. 7, the battery content 25a of the antenna unit 24a according to the second embodiment includes a positive electrode 20, a negative electrode 21, a gel electrolyte 22 which is formed by swelling of polymer with electrolytes, and a separator 23. The positive electrode 20 is a positive electrode of the battery 25. The negative electrode 21 is a negative electrode of the battery 25. The separator 23 is interposed between the positive electrode 20 and the negative electrode 21. The positive electrode 20 includes a positive-electrode power collector 20a and a positive-electrode active substance 20b. The negative electrode 21 includes a negative-electrode power collector 21a and a negative-electrode active substance 21b.

The gel electrolyte 22 and the separator 23 are stacked between the positive electrode 20 and the negative electrode 21 in layers. In this case, a layer of the separator 23 is formed on a side of the negative electrode 21. The positive-electrode active substance 20b is stacked between the positive-electrode power collector 20a and the gel electrolyte 22 in layers. The positive-electrode power collector 20a is stacked above the positive-electrode active substance 20b in layers. The negative-electrode active substance 21b is stacked between the negative-electrode power collector 21a and the separator 23 in layers. The negative-electrode power collector 21a is stacked below the negative-electrode active substance 21b in layers.

The battery content 25a including the multiple layers can be sheet-shaped. The battery content 25a is flexible and can be transformed easily according to the transformation of the battery outer covering 25b which is caused by the external acting force. When the battery outer covering 25b is transformed according to the movement of the subject 1, the battery content 25a can be transformed easily according to the transformation of the battery outer covering 25b.

The battery 25 in which the battery content 25a is sealed by the battery outer covering 25b is much thinner and lighter than a button-shaped battery, a dry-cell battery, and the like. The battery 25 attached to the body surface of the subject 1 can be flexibly transformed according to movement of the subject 1. The antenna unit 24a, in which the battery outer covering 25b covers the entire antenna unit, can be made further thinner lighter compared to a case where the outer covering of the entire antenna unit contains the battery, that is, a case where the battery outer covering contains the battery content.

As described above, in the second embodiment, the antenna unit can be easily transformed by the external acting force. The flexible outer covering seals the battery content, which provides a battery function, and is used as the battery covering. Mounted on the battery outer covering are the receiving antenna which receives the wireless signal from the capsule medical apparatus inside the subject, the transceiving circuit which converts the wireless signal received by the receiving antenna into the high-frequency signal, and the transmitting antenna which transmits the wireless signal which has been converted into the high-frequency signal by the transceiving circuit to the external medical receiving apparatus. The battery content is flexible and can be transformed easily according to transformation of the battery outer covering. Other configurations are the same as those of the first embodiment. Thus, the same operational effect as that of the first embodiment described can be provided, and, at the same time, a structure using a covering member or the like for fixating the battery itself on the flexible circuit substrate is no longer required. As a result, the antenna unit can be further made thinner and lighter compared to a case where the battery itself is mounted inside the outer covering.

A third embodiment is described. In the first embodiment described above, the transmitting antenna 12, the transceiving circuit 13, and the battery 14 are arranged on the flexible circuit substrate 10a, which is a part of the outer covering 10. The arranged positions in the first embodiment are outside the receiving antenna 11. In contrast, in the third embodiment, the transmitting antenna 12, the transceiving circuit 13, and the battery 14 are arranged inside the receiving antenna 12 on the flexible circuit substrate 10a.

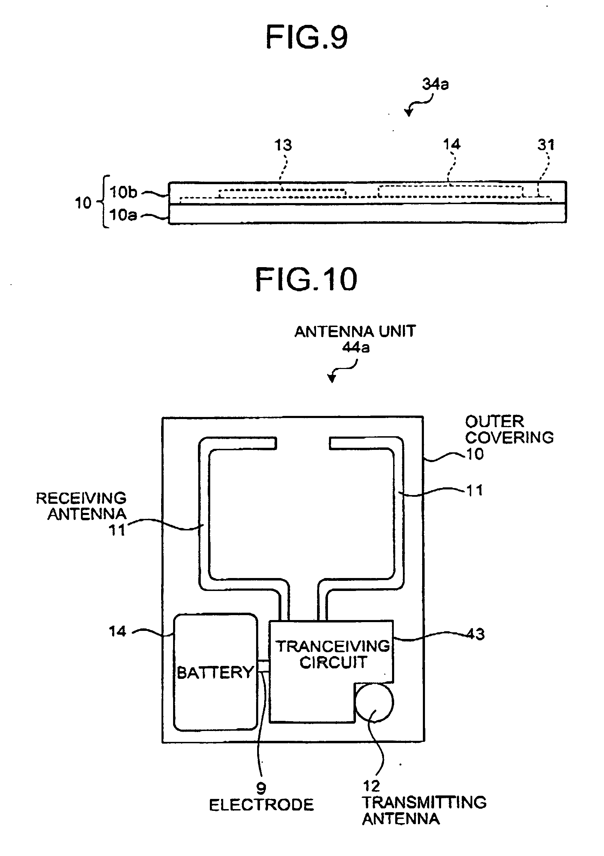

FIG. 8 is a schematic diagram of an exemplary configuration of an antenna unit according to the third embodiment. FIG. 9 is a schematic diagram of a side surface of the antenna unit shown in FIG. 8, viewed in direction A. In FIG. 8, the upper layer of the outer covering 10, i.e., the covering 10b, is omitted so that inner parts of the antenna unit according to the third embodiment can be more easily illustrated.

As shown in FIGS. 8 and 9, an antenna unit 34a according to the third embodiment includes a receiving antenna 31 instead of the receiving antenna 11 of the antenna unit 4a according to the first embodiment. In this case, the transmitting antenna 12, the transceiving circuit 13, and the battery 14 are arranged in inner space surrounded by the receiving antenna 31 on the flexible circuit substrate 10a. Other configurations are the same as those of the first embodiment, and the same numerals are attached to the same components.

Although not shown in the figures, an in-vivo information obtaining system according to the third embodiment includes antenna units 34a to 34h instead of the antenna units 4a to 4h of the in-vivo information obtaining system according to the first embodiment shown in FIG. 1. Of the antenna units 34a to 34h, the antenna unit 34a is described below. The remaining antenna units 34b to 34h have the same configuration as the antenna unit 34a.

The receiving antenna 31 is a flexible antenna made of a thin metal member such as a copper foil. As shown in FIG. 9, the receiving antenna 31 is arranged in the neighborhood of periphery of the flexible circuit substrate 10a, which is a part of the outer covering 10. The receiving antenna 31 forms as large opening area as possible within the flexible circuit substrate 10a. In this case, the opening area of the receiving antenna 31 is smaller than circuit area of the flexible circuit substrate 10a, and larger than area which can surround mounted area of the transmitting antenna 12, the transceiving circuit 13, and the battery 14. The receiving antenna 31 which forms the large opening area is more sensitive than the receiving antenna 11 according to the first embodiment described above, receives the wireless signal from the capsule medical apparatus 2 inside the subject 1 with high sensitivity, and transmits the received wireless signal to the transceiving circuit 13. The opening area of the receiving antenna 31 is area of opening space surrounded by an inner periphery of the receiving antenna 31.

As shown in FIG. 9, the transmitting antenna 12, the transceiving circuit 13, and the battery 14 of the antenna unit 34a according to the third embodiment are arranged inside the inner space surrounded by the receiving antenna 31 on the flexible circuit substrate 10a. It is preferable that the covering 10b, which covers the components, i.e., the receiving antenna 31, the transmitting antenna 12, the transceiving circuit 13, and the battery 14, mounted on the flexible circuit substrate, not contain metal so as to prevent deterioration of antenna characteristics of the receiving antenna 31. For example, it is preferable that the covering 10b be made of a flexible insulating member such as a sheet-shaped ceramic and resin. The resin member which forms the covering 10b is, for example, polyvinyl chloride, polypropylene, polyethylene, polyethylene terephthalate, polyester, and polyolefin.

As described above, in the third embodiment, the receiving antenna which receives the wireless signals from the capsule medical apparatus inside the subject is arranged near the periphery of the outer covering. Mounted in the inner substrate space surrounded by the receiving antenna are the transceiving circuit which converts the wireless signal received by the receiving antenna into the high-frequency signal, the transmitting antenna which transmits the wireless signal which has been converted into the high-frequency signal by the transceiving circuit to the medical receiving apparatus, and the battery which supplies power for the transceiving circuit. Other configurations are the same as those of the first embodiment. The opening area of the receiving antenna can be made as large as possible within the outer covering, whereby reception sensitivity of the receiving antenna can be further improved, and the inner space surrounded by the receiving antenna can be used effectively as the mounted space of the components such as the battery. Thus, the same operational effect as that of the first embodiment described above can be provided, and further, the antenna unit which has high sensitivity for receiving the wireless signal from the capsule medical apparatus inside the subject can be realized.

Further, because the inner space of the receiving antenna is used effectively as the mounted space of the components such as the battery, each of the components, such as the transceiving circuit and the battery, can be mounted inside the outer covering in a high density, whereby the antenna unit can be further downsized.

Further, because the components such as the battery are covered by the insulating member, the deterioration of the antenna characteristics of the receiving antenna is prevented even though the components such as the battery are mounted in the inner space surrounded by the receiving antenna.

A fourth embodiment is described. In the first embodiment described above, the frequency conversion is performed on the wireless signal transmitted from the capsule medical apparatus 2, and the wireless signal is relayed to the external medical receiving apparatus 3. In contrast, in the fourth embodiment, signal processes such as a demodulation process and a modulation process are performed on the wireless signal received from the capsule medical apparatus 2, and the wireless signal on which the signal processes have been performed is transmitted to the external medical receiving apparatus 3.

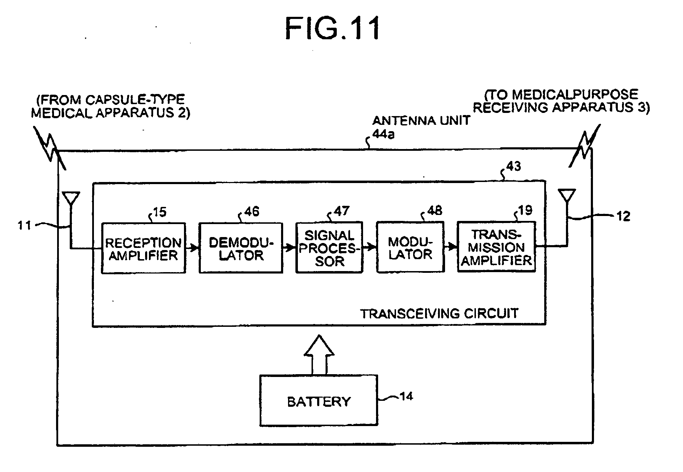

FIG. 10 is a schematic diagram of an exemplary configuration of an antenna unit according to the fourth embodiment. FIG. 11 is a schematic block diagram of an exemplary functional configuration of the antenna unit according to the fourth embodiment. In FIG. 10, the covering 10b which forms the upper layer of the outer covering 10 is omitted so that the inner parts of the antenna unit according to the fourth embodiment can be more easily illustrated.

As shown in FIGS. 10 and 11, an antenna unit 44a according to the fourth embodiment includes a transceiving circuit 43 instead of the transceiving circuit 13 of the antenna unit 4a according the first embodiment. The transceiving circuit 43 includes a demodulator 46, a signal processor 47, and a modulator 48 instead of the local oscillator 16, the frequency converter 17, and the bandwidth filter 18 of the transceiving circuit 13 described above. Other configurations are the same as those of the first embodiment, and the same numerals are attached to the same components.

Although not shown in the figures, an in-vivo information obtaining system according to the fourth embodiment includes antenna units 44a to 44h instead of the antenna units 4a to 4h of the in-vivo information obtaining system according to the first embodiment shown in FIG. 1. Of the antenna units 44a to 44h, the antenna unit 44a is described below. The remaining antenna units 44b to 44h have the same configuration as that of the antenna unit 44a.

The transceiving circuit 43 includes the demodulator 46, the signal processor 47, and the modulator 48 instead of the local oscillator 16, the frequency converter 17, and the bandwidth filter 18 of the transceiving circuit 13 of the antenna unit 4a according to the first embodiment. The transceiving circuit 43 receives the wireless signal from the capsule medical apparatus 2 via the receiving antenna 11. In each reception, the transceiving circuit 43 performs the signal processes such as the demodulation process and the modulation process on the received signal, and thus generates the wireless signal which can be received by the external medical receiving apparatus 3. The wireless signal generated by the transceiving circuit 43 includes the data of the in-vivo image of the subject 1 captured by the capsule medical apparatus 2. The transceiving circuit 43 transmits the generated wireless signal to the external medical receiving apparatus 3 via the transmitting antenna 12.

The demodulator 46 demodulates the received signal which is received from the capsule medical apparatus 2 by the receiving antenna 11, i.e., the wireless signal which includes the data of the in-vivo image of the subject 1. The demodulator 46 obtains the received signal from the capsule medical apparatus 2 which is amplified by the reception amplifier 15. The demodulator 46 performs the demodulation process and the like on the obtained received signal to thereby demodulate the received signal into a baseband signal. The demodulator 46 transmits the obtained baseband signal to the signal processor 47.

The signal processor 47 performs the predetermined signal processes on the baseband signal which is obtained through the demodulation by the demodulator 46. The signal processor 47 obtains the baseband signal which has been extracted through the demodulation process by the demodulator 46, and, based on the obtained baseband signal, generates the in-vivo images of the subject, i.e., the image signal which includes the image data captured by the capsule medical apparatus 2. Further, the signal processor 47 may perform a data compression process and the like on the image data of the generated image signal to thereby generate compressed data of the in-vivo image of the subject 1. When the image compression process is performed by the signal processor 47, the signal processor 47 transmits the image signal which includes the compressed data of the in-vivo image to the modulator 48.

The modulator 48 modulates the signal on which the signal process has been performed by the signal processor 47 to thereby generate the wireless signal which includes the data of the in-vivo image of the subject 1. The modulator 48 obtains the image signal which has been generated through the data compression process and the like by the signal processor 47 and performs the modulation process on the obtained image signal to thereby modulate the image signal into the wireless signal which can be received by the external medical receiving apparatus 3 described above. The wireless signal generated by the modulator 48 includes the compression data of the in-vivo image of the subject 1. The wireless signal is amplified by the transmission amplifier 19, then transmitted to the outside via the transmitting antenna 12 and received by the external medical receiving apparatus 3.

As described above, in the fourth embodiment, the signal which is received from the capsule medical apparatus via the receiving antenna is demodulated into the baseband signal. Based on the baseband signal, the signal processes such as the signal generation process for generating the image signal and the data compression process on the image signal are performed. The image signal on which the signal processes have been performed is modulated into the wireless signal, and the obtained wireless signal is transmitted to the external medical receiving apparatus via the transmitting antenna. Other configurations are the same as those of the first embodiment. Thus, the signal process, e.g., the data compression, which requires power, can be previously performed in the antenna unit before the wireless signal from the capsule medical apparatus is relayed to the external medical receiving apparatus. As a result, the same operational effect as that of the first embodiment described above can be provided, and the burden of the external medical receiving apparatus, which is caused by the signal process, can be reduced.

In the first, second, third, and fourth embodiments, all mounted parts on the flexible circuit substrate 10a are covered by the covering 10b, and all mounted parts on the lower-layer sealing member 26a are covered by the upper-layer sealing member 26b. Not limited to this, the configuration may be changed as long as the battery 14 at least is covered by the covering 10b of the outer covering described above, and the battery content 25a at least is covered by the upper-layer sealing member 26b of the battery outer covering 25b. When the covering 10b or the upper-layer sealing member 26b does not cover the receiving antennas 11, 31, nor the inside of these, the covering 10b or the upper-layer sealing member 26b may be made of a flexible conductive member which contains metals.

Further, in the first, second, and third embodiments, the wireless signal from the capsule medical apparatus 2 is converted into the high-frequency signal. Not limited to this, the wireless signal from the capsule medical apparatus 2 may be converted into a signal which has a lower frequency, and the wireless signal which has been converted into the low-frequency signal may be transmitted to the external medical receiving apparatus 3.

Further, in the fourth embodiment of the present invention, the signal processor 47 performs the signal generation process for generating the image signal based on the baseband signal which is obtained through the demodulation by the demodulator 46, and the data compression process for compressing the image signal. Not limited to this, the signal processor 47 may perform the signal generation process and further a code addition process for adding an error-correction code when there is an error in the image data. The signal processor 47 may perform at least one of the signal generation process, the data compression process, and the code addition process. The modulator 48 may modulates the image signal with a desired modulation method. Therefore, the modulator 48 may adopt a modulation method which is different from a modulation method used by the capsule medical apparatus 2. For example, when the capsule medical apparatus 2 adopts a binary modulation, the signal processor 47 may perform a multilevel modulation on the wireless signal on which the signal process has been performed by the signal processor 47.

Further, in the first, second, third, and fourth embodiments, the capsule medical apparatus 2 which obtains the in-vivo image as the in-vivo information of the subject 1 is described as an example, and the in-vivo image captured by the capsule medical apparatus 2 is relayed to the external medical receiving apparatus 3. Not limited to this, the in-vivo information of the subject 1 which is obtained by the capsule medical apparatus 2 may be measurement information such as a pH value and temperature inside the subject 1, or detection information of a body tissue inside the subject.

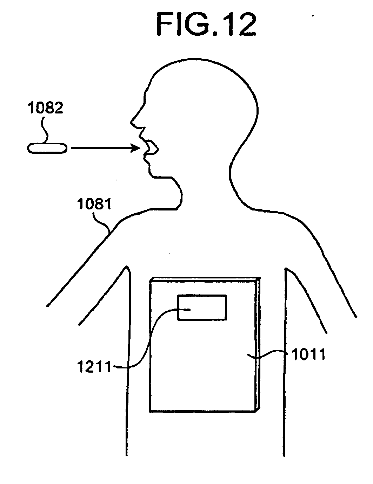

FIG. 12 shows an illustration of an attached medical receiving apparatus according to a fifth embodiment to which the present invention is applied.

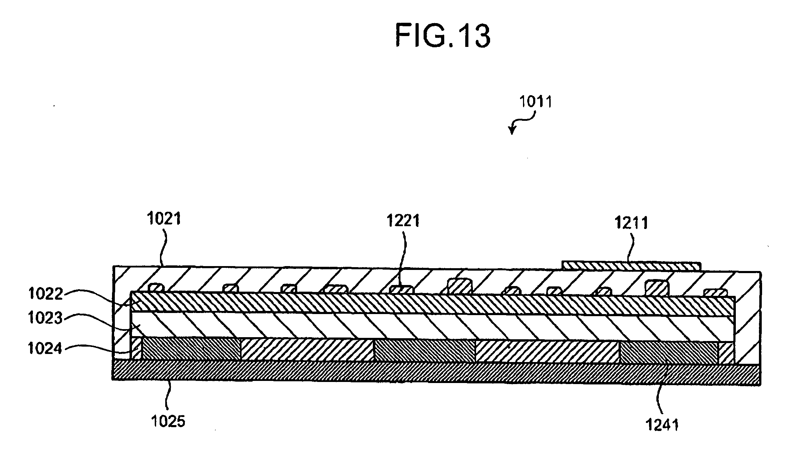

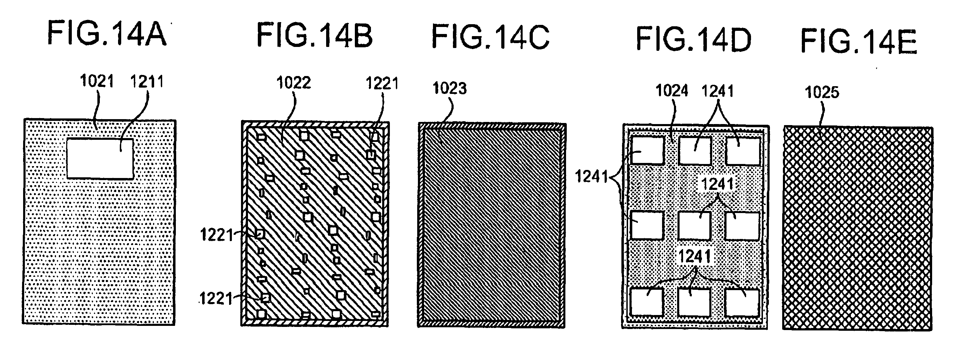

In FIG. 12, a capsule medical apparatus 1082 is swallowed from a mouth of a subject 1081, captures images while moving through the body cavity of the subject 1081, and, in each capturing, wirelessly transmits the captured image data in form of the wireless signal to a medical receiving apparatus 1011 which is located outside. The entire medical receiving apparatus 1011 is made of a flexible material so that the same can be directly attached on the subject 1081. The wireless signal, i.e., the image data, which is transmitted from the capsule medical apparatus 1082 is received by one of a plurality of receiving antennas 1241, which are described later in detail with reference to FIGS. 13 and 14A to 14E. After that, a flexible receiving-apparatus main unit performs predetermined processes, stores the processed data, and transfers the data to other apparatuses such as an external image processing apparatus and an image display workstation. When the processed data is transferred to the other apparatuses, the storing process of the processed data may be skipped. The transfer process of the processed data may be performed in each reception of the wireless signal transmitted from the capsule medical apparatus with the medical receiving apparatus 1011 attached on the subject 1081 or may be performed after the signal reception is completed. A method for the transfer process is not limited and can be a wired method and a wireless method (WLAN, UWB, Bluetooth, and the like).

FIG. 13 is a cross-sectional diagram of a side surface of the medical receiving apparatus according to the fifth embodiment to which the present invention is applied.

In FIG. 13, the medical receiving apparatus 1011 is of a size which can be easily attached on the subject 1081. For example, it is preferable that the medical receiving apparatus 1011 be around 300 mm in length, around 200 mm in width, and around 5 mm in thickness. The size in length and width is suitable for arranging the receiving antenna 1241 at a suitable position for receiving the image data transmitted from the capsule medical apparatus 1082 moving through the body cavity of the subject 1081. The size in thickness does not become in the way when the subject 1081 spends an ordinary life.

The medical receiving apparatus 1011 forms a four-layer structure in which a first layer is a flexible display device 1021, a second layer is a flexible receiving-apparatus main unit 1022, a third layer is a flexible power apparatus 1023, and a fourth layer is a flexible antenna apparatus 1024. The medical receiving apparatus 1011 may also form a five-layer structure which includes a flexible adhesive sheet 1025 as an additional fifth layer.

When the medical receiving apparatus 1011 forming the five-layer structure is attached on the subject 1081, the flexible adhesive sheet 1025 forms an outermost layer which touches the subject 1081 while the flexible display device 1021 another outermost layer which does not touch the subject 1081. Positions of the flexible receiving-apparatus main unit 1022 and the flexible power apparatus 1023 may be swapped. A part or parts of the flexible antenna apparatus 1024 may be arranged in the same plane as the flexible receiving-apparatus main body 1022 or the flexible power apparatus 1023 which is arranged closer to the body surface of the subject 1081, or in the same plane with both. In this case, the medical receiving apparatus may form a three-layer structure.

Further, when the medical receiving apparatus 1011 forming the four-layer structure is attached on the subject 1081, the flexible antenna apparatus 1024 forms an outermost layer which touches the subject 1081. Similarly to the medical receiving apparatus 1011 forming the five-layer structure, the flexible display device 1021 forms another outermost layer which does not touch the subject 1081. The four-layer medical receiving apparatus 1011 does not require an adhesive sheet, e.g., the flexible adhesive sheet 1025, and can be put onto the subject 1081 to be attached thereon. Further, when the adhesive sheet like the flexible adhesive sheet 1025 is attached to a side of the flexible antenna apparatus 1024 of the four-layer medical receiving apparatus 1011, the four-layer medical receiving apparatus 1011 can be equipped similarly to the five-layer medical receiving apparatus 1011. Similarly to the five-layer medical receiving apparatus 1011, in the four-layer medical receiving apparatus 1011, the positions of the flexible receiving-apparatus main unit 1022 and the flexible power apparatus 1023 can be swapped.

FIGS. 14A to 14E are schematic diagrams of the medical receiving apparatus according to the fifth embodiment to which the present invention is applied. Specifically, the FIG. 14A shows the flexible display device, FIG. 14B shows the flexible receiving-apparatus main unit 1022, FIG. 14C shows the flexible power apparatus 1023, FIG. 14D shows the flexible antenna apparatus 1024, and FIG. 14E shows the flexible adhesive sheet 1025.

The flexible display device 1021 is called an electronic paper and, literally, a display device which is thin and flexible like a paper. The flexible display device 1021 is suitable for the outer layer of the medical receiving apparatus. The flexible display device 1021 can display digital information such as documents and images used by computers on a display unit 1211. The flexible display device 1021 is usually light-reflective and is easy to view and handle like a paper. The flexible display device 1021 may be of various types such as an electrophoretic type and a heat-sensitive type. The flexible display device 1021 can perform a colored presentation with an additional color filter. The entire flexible display device 1021 except the display unit 1211 is made of a soft resin such as an elastomer resin so that the flexible display device 1021 can be used as the outer covering of the medical receiving apparatus 1011. The medical receiving apparatus 1011 whose outer covering is made of the soft resin has a high level of shock absorption and thus does not break down easily due to shock caused by falling or the like.

The flexible receiving-apparatus main unit 1022 shown in FIG. 14B is thin and flexible with high flexible characteristics. In the flexible receiving-apparatus main unit 1022, a copper foil is bonded with a flexible base file made of polyimide resin, polyethylene terephthalate (PET) resin, or the like to form a copper multilayer plate. Then, a wiring is formed by etching other portions of the copper foil of the copper multilayer plate, whereby a pattern circuits 1221 such as a digital signal processing circuit, a memory, and an RF circuit are formed. The pattern circuits 1221 include a signal processing circuit which is required for processing the image data, a storage circuit which is a required component for transferring the demodulated signal to other apparatuses such as the external image processing apparatus and the external image display workstation, a rectifying circuit which is a required component for noncontact power supply, and a wireless-signal generating circuit which is a required component for noncontact transfer. Since the data can be transmitted to other apparatuses on the noncontact transfer, medical workers field can observe the subject 1081 in real time using a personal computer or the like while the subject 1081 can act freely. Further, since power can be supplied from the flexible power apparatus 1023 on the noncontact power supply, a low-cost waterproof system can be realized.

The pattern circuits 1221 such as the digital signal processing circuit and the like can be made thin and small using COB (Chip On Board) implementation technology and the like. A protection sheet, which includes an adhesive layer formed on a coverlay film, is stacked on the pattern circuits 1221 as an inner layer, and the adhesive is hardened.

When the flexible receiving-apparatus main unit needs to include a rigid member whose area is one-sixtieth of or larger than that of the flexible receiving-apparatus main unit, the rigid member can be arranged in the periphery of the flexible receiving-apparatus main unit 1022. Specifically, when the flexible receiving-apparatus main unit needs to include a large component such as CompactFlash (registered trademark), which occupies a large area, the large component is arranged not in the center but in the periphery of the flexible receiving-apparatus main unit 1022, whereby the medical receiving apparatus 1011 can maintain its flexibility easily. The area of such a component is described as sixtieth of or larger than that of the flexible receiving-apparatus main unit 1022, so that the component can be easily arranged near the body surface of the subject 1081. For example, when the size of the medical receiving apparatus 1011 is A4, the medical receiving apparatus 1011 can include an apparatus which is of the size of CompactFlash (registered trademark).

The flexible power apparatus 1023 shown in FIG. 14C, for example, includes three stacked layers in which an electrolyte sheet is sandwiched by the positive electrode sheet and the negative electrode sheet which contain active substances. Further, in the flexible power apparatus 1023, a positive-electrode power collection plate and a negative-electrode power collection plate are stacked in layers on the positive electrode sheet and the negative electrode sheet. These plates make potential distribution on the positive electrode sheet and the negative electrode sheet even, and obtain current from potential difference between the positive electrode sheet and the negative electrode sheet. The stacked-layers unit including the stacked layers above is sealed by a flexible package sheet, whereby the sheet-shaped flexible power apparatus 1023 is formed. Portions of the positive-electrode and negative-electrode power collecting plates which are exposed from the package sheet are used as terminals of the flexible power apparatus 1023, and desired amount of power can be thus obtained.

The flexible antenna apparatus 1024 shown in FIG. 14D is sheet-shaped and made of a soft material similarly to the flexible display device 1021, the flexible receiving-apparatus main unit 1022, and the flexible power apparatus 1023 described above. The flexible antenna apparatus 1024 includes at least one receiving antenna 1241, which is a receiving power antenna circuit formed on the flexible substrate as a wiring antenna. For example, the flexible antenna apparatus 1024 includes nine receiving antennas 1241. The flexible antenna apparatus 1024 receives, via one of the receiving antennas 1241, the wireless signal transmitted from the capsule medical apparatus 1082. The flexible antenna apparatus 1024 transmits the wireless signal to the flexible receiving-apparatus main unit 1022.

The flexible display device 1021, the flexible receiving-apparatus main unit 1022, the flexible power apparatus 1023, and the flexible antenna apparatus 1024 are electrically connected with each other. Specifically, the flexible display device 1021, the flexible receiving-apparatus main unit 1022 are operated by power supplied from the flexible power apparatus 1023. Image data is received by one of the receiving antennas 1241 in the flexible receiving-apparatus main unit 1022, and then processed by the digital signal processing circuit and the like in the flexible receiving-apparatus main unit 1022. After that, a result of the process is displayed on the display unit 1211 in the flexible display device 1021.

It is preferable that the data transfer method for transferring data from the flexible receiving-apparatus main unit 1022 to other apparatuses such as the external image processing apparatus and the image display workstation be noncontact transfer via wireless transmission, e.g., WLAN, UWB, and Bluetooth. To achieve this, the wireless-signal generating circuit is required as a component. Further, it is preferable that the power supply from the flexible power apparatus 1023 be noncontact power supply. To achieve this, the rectifying circuit is required as a component.

The noncontact transfer and noncontact power supply do not require connection points for signal transfer and power supply on the outer covering of the flexible receiving-apparatus main unit 1022, whereby the low-cost waterproof system can be realized. Further, the noncontact transfer allows medical workers to observe the subject 1081 in real time using a personal computer or the like while the subject 1081 can act freely.

The flexible adhesive sheet 1025 shown in FIG. 14E is a two-sided adhesive sheet which has an adhesive layer formed on both sides of a main sheet thereof, and can be peeled off. The adhesive layer of the adhesive sheet is made of a reacted substance between a hardening agent and adhesive polymer. The hardening agent has an isocyanate group consisting of ring polymer of di-isocyanate, and the adhesive polymer has an active-hydrogen-containing group. By the flexible adhesive sheet 1025, the flexible antenna apparatus 1024 can be adhered to the subject 1081, temporarily, while the subject is observed (examined).

The medical receiving apparatus 1011 according to the fifth embodiment described above is flexible, and attached to the body surface (i.e., abdominal surface) of the subject 1081 when in use. Therefore, the medical receiving apparatus 1011 is transformed according to stretching of the body surface, and always adhered thereto.

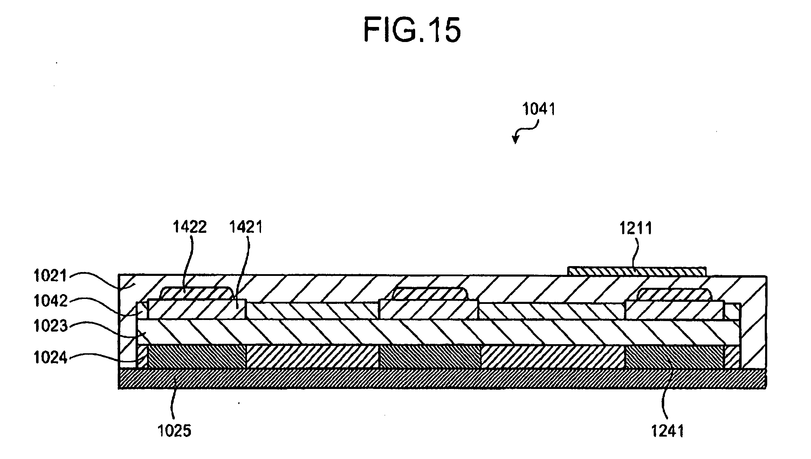

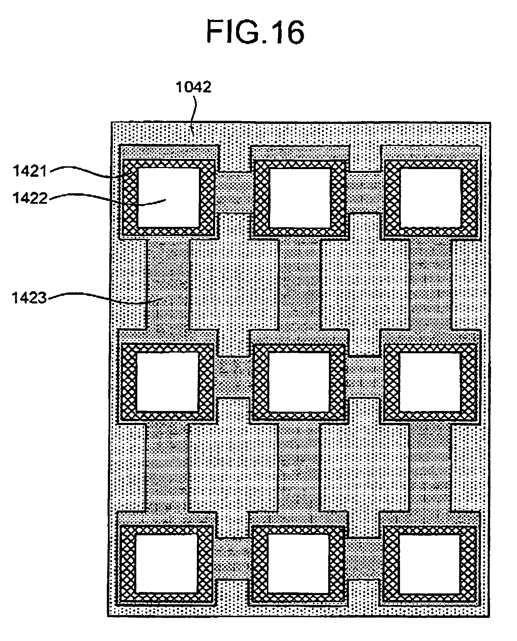

FIG. 15 is a cross-sectional diagram which views a side surface of a medical receiving apparatus according to a sixth embodiment to which the present invention is applied. FIG. 16 shows a flexible receiving-apparatus main unit according to the sixth embodiment to which the present invention is applied.

Similarly to the medical receiving apparatus 1011, a medical receiving apparatus 1041 shown in FIG. 15 is of a size which can be easily attached on the subject 1081. Further, similarly to the medical receiving apparatus 1011, the medical receiving apparatus 1041 forms a four-layer structure in which the first layer is flexible display device 1021, the third layer is the flexible power apparatus 1023, the fourth layer is the flexible antenna apparatus 1024, and the second layer is a flexible receiving-apparatus main unit 1042 instead of the flexible receiving apparatus main unit 1022. The medical receiving apparatus 1041 may also form a five-layer structure with an additional fifth layer of the flexible adhesive sheet 1025.

The flexible receiving-apparatus main unit 1042 is thin and flexible with high flexible characteristics similarly to the flexible receiving-apparatus main unit 1022.

In the fifth embodiment, the pattern circuits 1221 such as the digital signal processing circuit, the memory, and the RF circuit are arranged across the entire flexible receiving-apparatus main unit 1022. In contrast, in the flexible receiving-apparatus main unit 1042 according to the sixth embodiment, the pattern circuits 1221 such as the digital signal processing circuit, the memory, and the RF circuit are mounted on the circuit mounted area 1422 as circuit units 1421. The flexible receiving-apparatus main unit 1042 includes more than one circuit units 1421, which are divided into several groups, unlike the fifth embodiment. The circuit units 1421 are not flexible, and connected with each other via a flexible cable 1423 made of multiple conductive members and flexible insulating materials.

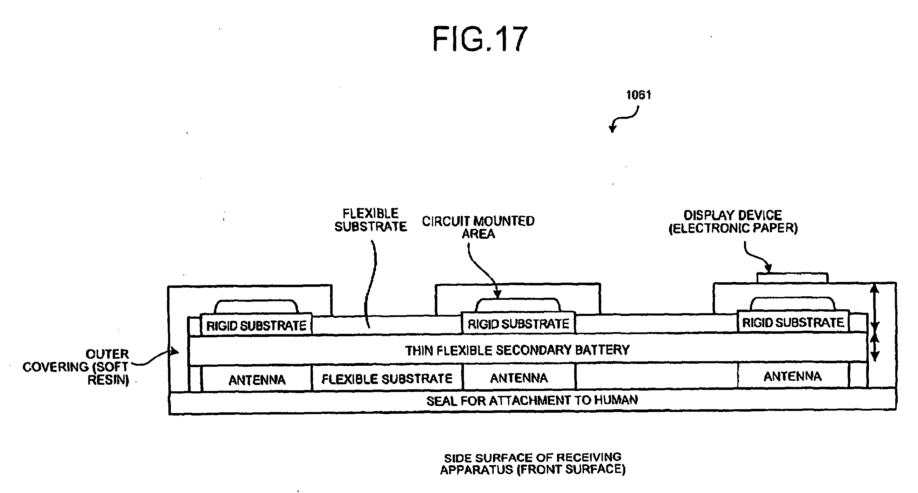

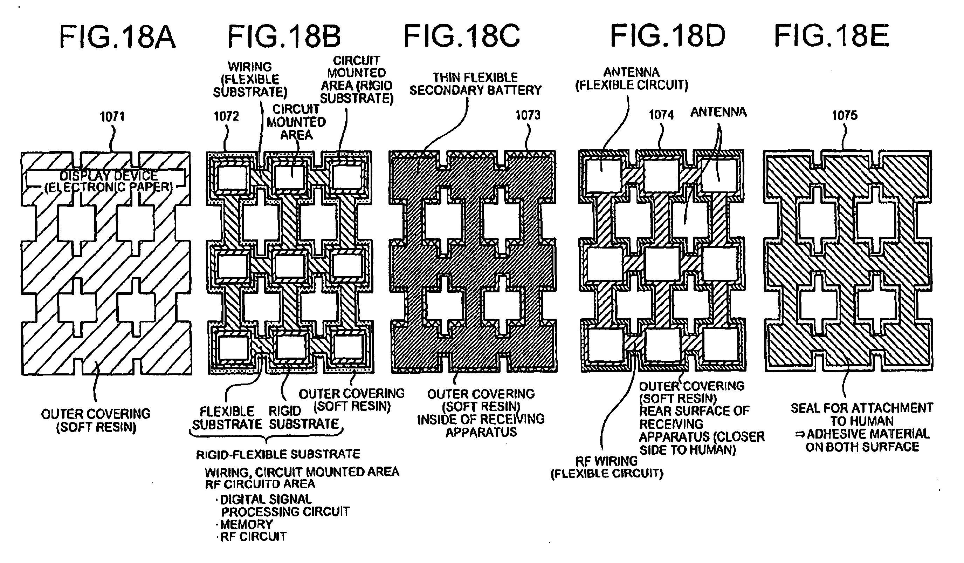

FIG. 17 is a cross-sectional diagram which views a side surface of a medical receiving apparatus according to a seventh embodiment to which the present invention is applied. FIGS. 18A to 18E are schematic diagrams of medical receiving apparatus according to the seventh embodiment to which the present invention is applied.

In FIG. 17, a medical receiving apparatus 1061 is of a size which can be easily attached on the subject 1081, similarly to the medical receiving apparatus 1011. As shown in FIGS. 18A to 18E, the medical receiving apparatus 1061 forms a four-layer structure in which the first layer is a flexible display device 1071 (FIG. 18A), the second layer is the flexible receiving-apparatus main unit 1072 (FIG. 18B), the third layer is a flexible power apparatus 1073 (FIG. 18C), and the fourth layer is a flexible antenna apparatus 1074 (FIG. 18D). The medical receiving apparatus 1061 may also form a five-layer structure with an additional fifth layer of a flexible adhesive sheet 1075 (FIG. 18E).

The medical receiving apparatus 1061 is thin, and each layer thereof forms a mesh structure, so that the medical receiving apparatus 1061 is more flexible with higher flexible characteristics than the medical receiving apparatus 1011. Therefore, the medical receiving apparatus 1061 can be attached on the subject 1081 more easily. The medical receiving apparatus 1061 can suppress loss of received power and improve resistance against external noise. Further, when the flexible adhesive sheet 1075 is used as the fifth layer, the flexible antenna apparatus 1074 can adhere to the body surface of the subject 1081 more easily than when the flexible adhesive sheet 1075 is not used, and can thus suppress loss of power for reception, and improve resistance against external noise more effectively.

The antenna units according to the embodiments include the thin, flexible power supply unit and relay the wireless signal from the capsule medical apparatus within the subject to the external receiving apparatus without using cables. Therefore, the antenna units can be made thin and light, and the cables for connecting the antenna units with the external receiving apparatus are not required. As a result, the antenna units can reduce burden on the subject while receiving the in-vivo information obtained by the capsule medical apparatus inside the subject and can be easily arranged on the subject.

In the medical receiving apparatus according to the embodiments, the receiving antenna and the receiving-apparatus main unit are flexible and are thus not in the way when the medical receiving apparatus is attached on the subject. Further, the medical receiving apparatus transfers data to other external apparatuses such as the image processing apparatus and the image display workstation on the noncontact transfer, whereby the medical worker can observe the subject in real time using the personal computer or the like while the subject can act freely.

In the medical receiving apparatus according to the embodiments, the receiving antenna and the receiving-apparatus main unit are flexible, and can thus be integrated together. Therefore, wires for electrically connecting the receiving antenna with the flexible receiving-apparatus main unit can be short. As a result, the medical receiving apparatus which suppresses loss of power for reception and improves resistance against external noise can be realized. Further, since power is supplied from the flexible power apparatus on noncontact, the low-cost waterproof system can be realized.

The medical receiving apparatus according to the embodiments are made of a soft resin having a high level of shock absorption, and thus does not break down easily due to shock caused by falling or the like.

## Claims

1. A receiving apparatus (1011; 1041; 1061) for a capsule medical apparatus (2), comprising:
a flexible antenna apparatus (1024; 1074) including a flexible sheet and at least one receiving antenna (1241) adapted to receive a wireless signal transmitted from an external capsule medical apparatus (1082), the receiving antenna (1241) being arranged in two dimensions;
a sheet-shaped flexible receiving-apparatus main unit (1022; 1042; 1072) on which a demodulation circuit adapted to convert the wireless signal received by the receiving antennas (1241) into a baseband signal is arranged;
a sheet-shaped flexible power apparatus (1023; 1073) adapted to supply power for the demodulation circuit;
**characterized in that** the receiving apparatus further comprises
a sheet-shaped flexible display device (1021; 1071) which uses power supplied from the flexible power apparatus (1023; 1073), and comprises a display unit (1211) which displays information related to the received wireless signal, wherein
the flexible antenna apparatus (1024; 1074), the flexible receiving-apparatus main unit (1022; 1042; 1072), the flexible power apparatus (1023; 1073), and the sheet-shaped flexible display device (1021; 1071) are electrically connected with each other in a layer structure wherein the flexible antenna apparatus (1024; 1074) forms an outermost layer which touches the subject (1081) in case the receiving apparatus (1011; 1041; 1061) is in use.

2. The receiving apparatus (1011; 1041; 1061) according to claim 1, wherein
the at least a part of the flexible antenna apparatus (1024; 1074) is arranged in a same plane with at least one of the flexible receiving-apparatus main unit (1022; 1042; 1072) and the flexible power apparatus (1023; 1073).

3. The receiving apparatus (1061) according to claims 1 or 2, wherein
the flexible antenna apparatus (1074), the flexible receiving-apparatus main unit (1072), and the flexible power apparatus (1073) are mesh-shaped.

4. The receiving apparatus (1011; 1041; 1061) according to any one of claims 1 to 3, wherein
a flexible adhesive sheet (1025; 1075) is arranged so as to cover the outermost layer.

5. The receiving apparatus (1011; 1061) according to any one of claims 1 to 4, wherein
the flexible receiving-apparatus main unit (1022; 1042; 1072) includes at least one of a signal processing circuit, a memory circuit, a rectifying circuit, and a wireless-signal generating circuit.

6. The receiving apparatus (1011; 1041; 1061) according to any one of claims 1 to 5, wherein
when the flexible receiving-apparatus main unit (1022; 1042; 1072) needs to include a rigid member whose area is one-sixtieth of or larger than that of the flexible receiving-apparatus main unit (1022; 1042; 1072), the rigid member is arranged on the periphery of the flexible receiving-apparatus main unit (1022; 1042; 1072).

7. The receiving apparatus (1011; 1041; 1061) according to any one of claims 1 to 6, wherein
a portion other than at least one of the flexible antenna apparatus (1024; 1074), the flexible adhesive sheet (1025; 1075), and the flexible display device (1021; 1071) is coated with a soft resin.

8. The receiving apparatus (1011; 1041; 1061) according to claim 7, wherein the soft resin is an elastomer resin.

## Patentansprüche

1. Empfangseinrichtung (1011; 1041; 1061) für eine kapselförmige medizinische Einrichtung (2), die umfasst:
- eine flexible Antenneneinrichtung (1024; 1074), die eine flexible Platte und wenigstens eine Empfangsantenne (1241) umfasst, die dazu eingerichtet ist, ein drahtloses Signal zu empfangen, das von einer externen kapselförmigen medizinischen Einrichtung (1082) gesendet wird, wobei die Empfangsantenne (1241) in zwei Dimensionen angeordnet ist,
- eine plattenförmige flexible Empfangseinrichtungshaupteinheit (1022; 1042; 1072), an der eine Demodulationsschaltung angeordnet ist, die dazu eingerichtet ist, das von den Empfangsantennen (1241) empfangene drahtlose Signal in ein Basisbandsignal umzuwandeln,
- eine plattenförmige flexible Stromversorgungseinrichtung (1023; 1073), die dazu eingerichtet ist, die Demodulationsschaltung mit Strom zu versorgen,
**dadurch gekennzeichnet, dass** die Empfangseinrichtung ferner umfasst:
- eine plattenförmige flexible Anzeigeeinrichtung (1021; 1071), die von der flexiblen Stromversorgungseinrichtung (1023; 1073) zugeführten Strom verwendet und eine Anzeigeeinheit (1211) umfasst, die auf das empfangene drahtlose Signal bezogene Informationen anzeigt, wobei
- die flexible Antenneneinrichtung (1024; 1074), die flexible Empfangseinrichtungshaupteinheit (1022; 1042; 1072), die flexible Stromversorgungseinrichtung (1023; 1073) und die plattenförmige flexible Anzeigeeinrichtung (1021; 1071) in einer Schichtstruktur elektrisch miteinander verbunden sind, wobei die flexible Antenneneinrichtung (1024; 1074) eine äußerste Schicht bildet, die die Person (1081) berührt, wenn die Empfangseinrichtung (1011; 1041; 1061) in Gebrauch ist.

2. Empfangseinrichtung (1011; 1041; 1061) nach Anspruch 1, wobei wenigstens ein Teil der flexiblen Antenneneinrichtung (1024; 1074) mit der flexiblen Empfangseinrichtungshaupteinheit (1022; 1042; 1072) und/oder der flexiblen Stromversorgungseinrichtung (1023; 1073) in derselben Ebene angeordnet ist.

3. Empfangseinrichtung (1061) nach Anspruch 1 oder 2, wobei die flexible Antenneneinrichtung (1074), die flexible Empfangseinrichtungshaupteinheit (1072) und die flexible Stromversorgungseinrichtung (1073) gitterförmig ausgeführt sind.

4. Empfangseinrichtung (1011; 1041; 1061) nach einem der Ansprüche 1 bis 3, wobei eine flexible Haftplatte (1024; 1075) derart angeordnet ist, dass sie die äußerste Schicht bedeckt.

5. Empfangseinrichtung (1011; 1061) nach einem der Ansprüche 1 bis 4, wobei die flexible Empfangseinrichtungshaupteinheit (1022; 1042; 1072) eine Signalverarbeitungsschaltung, eine Speicherschaltung, eine Gleichrichterschaltung und/oder eine Erzeugungsschaltung für drahtlose Signale umfasst.

6. Empfangseinrichtung (1011; 1041; 1061) nach einem der Ansprüche 1 bis 5, wobei, wenn die flexible Empfangseinrichtungshaupteinheit (1022; 1042; 1072) ein starres Element enthalten muss, dessen Fläche ein Sechzigstel der flexiblen Empfangseinrichtungshaupteinheit (1022; 1042; 1072) oder mehr beträgt, das starre Element am Umfang der flexiblen Empfangseinrichtungshaupteinheit (1022; 1042; 1072) angeordnet ist.

7. Empfangseinrichtung (1011; 1041; 1061) nach einem der Ansprüche 1 bis 6, wobei ein Abschnitt, der nicht die flexible Antenneneinrichtung (1024; 1074), die flexible Haftplatte (1025; 1075) und/oder die flexible Anzeigeeinrichtung (1021; 1071) einschließt, mit einem Weichharz beschichtet ist.

8. Empfangseinrichtung (1011; 1041; 1061) nach Anspruch 7, wobei das Weichharz ein Elastomerharz ist.

## Revendications

1. Appareil de réception (1011 ; 1041 ; 1061) pour un appareil médical (2) de type capsule, comprenant :
un appareil (1024 ; 1074) d'antenne flexible incluant une feuille flexible et au moins une antenne de réception (1141) adaptée pour recevoir un signal sans fil transmis depuis un appareil médical externe (1082) de type capsule, l'antenne de réception (1241) étant disposée en deux dimensions ;
une unité principale (1022 ; 1042 ; 1072) d'appareil de réception flexible en forme de feuille sur laquelle un circuit de démodulation adapté pour convertir le signal sans fil reçu par les antennes de réception (1241) dans un signal de bande de base est agencée ;
un appareil (1023 ; 1073) de puissance flexible en forme de feuille adapté pour délivrer la puissance pour le circuit de démodulation ;
**caractérisé en ce que** l'appareil de réception comprend en outre
un dispositif (1021 ; 1071) d'affichage flexible en forme de feuille qui utilise la puissance délivrée depuis l'appareil (1023 ; 1073) de puissance flexible, et comprend une unité d'affichage (1211) qui affiche des informations se rapportant au signal sans fil reçu, dans lequel
l'appareil (1024 ; 1074) d'antenne flexible, l'unité principale (1022 ; 1042 ; 1072) d'appareil de réception flexible, l'appareil (1023 ; 1073) de puissance flexible, et le dispositif (1021 ; 1071) d'affichage flexible en forme de feuille sont électriquement connectés les uns aux autres dans une structure en couches dans laquelle l'appareil (1024 ; 1074) d'antenne flexible forme la couche la plus à l'extérieur qui touche le sujet (1081) dans un cas dans lequel l'appareil de réception (1011 ; 1041 ; 1061) est en utilisation.

2. Appareil de réception (1011 ; 1041 ; 1061) selon la revendication 1, dans lequel
la au moins une partie de l'appareil (1024 ; 1074) d'antenne flexible est disposée dans un même plan avec au moins l'un parmi l'unité principale (1022 ; 1042 ; 1072) d'appareil de réception flexible et l'appareil (1023 ; 1073) de puissance flexible.

3. Appareil de réception (1061) selon la revendication 1 ou 2, dans lequel
l'appareil (1074) d'antenne flexible, l'unité principale (1072) d'appareil de réception flexible, et l'appareil (1073) de puissance flexible sont en forme de mailles.

4. Appareil de réception (1011 ; 1041 ; 1061) selon l'une quelconque des revendications 1 à 3, dans lequel
une feuille adhésive flexible (1025 ; 1075) est disposée de façon à couvrir la couche la plus à l'extérieur.

5. Appareil de réception (1011 ; 1061) selon l'une quelconque des revendications 1 à 4, dans lequel
l'unité principale (1022 ; 1042 ; 1072) d'appareil de réception flexible comprend au moins l'un parmi un circuit de traitement de signal, un circuit de mémoire, un circuit redresseur et un circuit de génération de signal sans fil.

6. Appareil de réception (1011 ; 1041 ; 1061) selon l'une quelconque des revendications 1 à 5, dans lequel
lorsque l'unité principale (1022 ; 1042 ; 1072) d'appareil de réception flexible doit inclure un élément rigide dont la superficie représente un soixantième ou plus de celle de l'unité principale (1022 ; 1042 ; 1072) d'appareil de réception flexible, l'élément rigide est agencé sur la périphérie de l'unité principale (1022 ; 1042 ; 1072) d'appareil de réception flexible.

7. Appareil de réception (1011 ; 1041 ; 1061) selon l'une quelconque des revendications 1 à 6, dans lequel
une partie autre qu'au moins l'un parmi l'appareil (1024 ; 1074) d'antenne flexible, la feuille adhésive flexible (1025 ; 1075), et le dispositif (1021 ; 1071) d'affichage flexible est revêtue d'une résine molle.

8. Appareil de réception (1011 ; 1041 ; 1061) selon la revendication 7, dans lequel la résine molle est une résine élastomère.
